(19) Europäisches Patentamt
European Patent Office
Office européen des brevets

(11) Publication number: **0 039 089**
**B1**

(12) **EUROPEAN PATENT SPECIFICATION**

(45) Date of publication of patent specification: **05.10.83**

(21) Application number: **81103288.7**

(22) Date of filing: **30.04.81**

(51) Int. Cl.³: **C 07 C 45/67,** C 07 C 49/707, C 07 C 49/743, C 07 C 49/747, C 07 C 49/723

(54) **Process for preparing cyclopentenolones.**

(30) Priority: **30.04.80 JP 58475/80**
**10.06.80 JP 78528/80**
**22.10.80 JP 148530/80**
**20.11.80 JP 164372/80**

(43) Date of publication of application:
**04.11.81 Bulletin 81/44**

(45) Publication of the grant of the patent:
**05.10.83 Bulletin 83/40**

(84) Designated Contracting States:
**BE CH DE FR GB IT LI NL SE**

(56) References cited:
**EP - A - 0 022 162**

**CHEMICAL ABSTRACTS, vol. 89, no. 15, October 9, 1978, page 554, abstract 129107g, COLOMBUS, OHIO (US)**

**CHEMICAL ABSTRACTS, vol. 85, 1976, page 514, abstract 7759s, COLOMBUS, OHIO (US)**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Saito, Kenji**
**2-10-3-321, Sonehigashi-machi**
**Toyonaka Osaka (JP)**
Inventor: **Yamachika, Hiroshi**
**332, Sumitomo Kagaku Apartment 2-1, Kuwata-cho**
**Ibaraki, Osaka (JP)**
Inventor: **Minai, Masayoshi**
**1606-5, Harimada-cho**
**Moriyama, Shiga (JP)**

(74) Representative: **Vossius, Dorothea et al,**
**VOSSIUS VOSSIUS TAUCHNER HEUNEMANN RAUH P.O. Box 86 07 67 Siebertstrasse 4**
**D-8000 Munich 86 (DE)**

(56) References cited:
**INTERNATIONAL JOURNAL OF METHODS IN SYNTHETIC ORGANIC CHEMISTRY, no. 2, 1977, G. PIANCATELLI et al.: "A simple conversion of 4-substituted-5-hydroxy-3 oxo-cyclopentenes into the 2-substituted Analogs", pages 116, 117**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 039 089 B1

Process for preparing cyclopentenolones

The present invention relates to a process for preparing cyclopentenolones. More particularly, it relates to a novel and improved process for preparing 2—$R_1$—3—$R_2$—4-hydroxy-2-cyclopentenones of the formula:

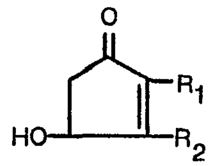

(I)

wherein $R_1$ is an alkyl group having not more than 6 carbon atoms, an alkenyl group having not more than 6 carbon atoms, an alkynyl group having not more than 6 carbon atoms or a group of the formula:

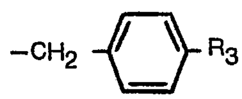

(in which $R_3$ is hydrogen, methyl or halogen) and $R_2$ is a hydrogen atom or a methyl group, provided that when $R_2$ is hydrogen, $R_1$ is neither $\alpha$-methylallyl nor $\alpha$-methylpropargyl.

In the above significances, the terms "alkyl", "alkenyl" and "alkynyl" are intended to mean straight, branched and cyclic ones, inclusively. Specific examples of them are methyl, ethyl, n-propyl, isopropyl, n-butyl, isobutyl, sec-butyl, n-pentyl, n-hexyl, allyl, $\alpha$-methylallyl, $\alpha$-ethylallyl, 4-pentenyl, propargyl, $\alpha$-methylpropargyl, cyclopentyl, cyclohexyl, 2-cyclopentenyl, 2-cyclohexenyl, etc. The term "halogen" is intended to mean chlorine, bromine, fluorine, etc.

The 2—$R_1$—3—$R_2$—4-hydroxy-2-cyclopentenones (I) are useful as the alcoholic components of agricultural chemicals such as allethrines. For their production, various methods are known, some of which are used in industry. But they are still not satisfactory in respect of the yield, the complexity of operations, the problems of environmental pollution, etc.

For preparation of the 2—$R_1$—3—$R_2$—4-hydroxy-2-cyclopentenones (I), there are known three procedures starting from the corresponding 4-hydroxy-4—$R_2$—5—$R_1$—2-cyclopentenones of the formula:

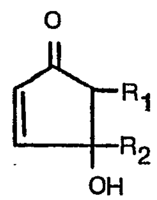

(II)

wherein $R_1$ and $R_2$ are each as defined above, of which the one comprises treating the 4-hydroxy-4—$R_2$—5—$R_1$—2-cyclopentenones (II) with basic alumina in a solvent mixture of benzene and ether [G. Piancatelli et al.: Tetrahedron, *34,*, 2775 (1978)], the second comprises treating the 4-hydroxy-4—$R_2$—5—$R_1$—2-cyclopentenones (II) with 5% sodium hydrogen carbonate [Japanese Patent Publication (unexamined) No. 21146/78], while the third procedure is characterized by the treatment of the compounds (II) with a base [EU—A—0 022 162, unpublished]. However, the first procedure is economically disadvantageous in requiring a large amount of expensive alumina. The second procedure necessitates strong agitation for uniform dispersion of the 4-hydroxy-4—$R_2$—5—$R_1$—2-cyclopentenones (II) into the sodium hydrogen carbonate solution and is not satisfactory in the yield of the objective 2—$R_1$—3—$R_2$—4-hydroxy-2-cyclopentenones (I).

On the other hand, it is known that treatment of a prostaglandin intermediate of the formula:

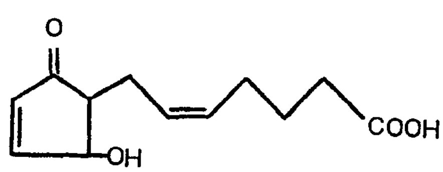

with 1N sulfuric acid in a solvent mixture of water and dioxane affords a prostaglandin of the formula:

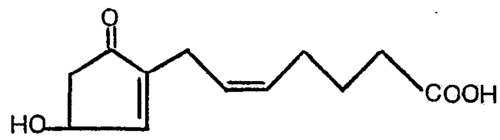

[M. B. Floyd, J. Org. Chem., *43,* 1641 (1978)]. When the reaction conditions in this treatment are applied to the conversion of the 4-hydroxy-4—$R_2$—5—$R_1$—2-cyclopentenones (II) into the 2—$R_1$—3—$R_2$—4-hydroxy-2-cyclopentenones (I), various undesired substances result as by-products, and yields of the objective 2—$R_1$—3—$R_2$—4-hydroxy-2-cyclopentenones (I) are very greatly lowered.

As the result of an extensive study, it has now been found that treatment of the 4-hydroxy-4—$R_2$—5—$R_1$—2-cyclopentenones (II) with an aqueous medium affords readily the corresponding 2—$R_1$—3—$R_2$—4-hydroxy-2-cyclopentenones (I) in a good yield.

According to the present invention, there is provided a process for preparing the 2—$R_1$—3—$R_2$—4-hydroxy-2-cyclopentenones (I) in a good yield within a short period of time which comprises treatment of the corresponding 4-hydroxy-4—$R_2$—5—$R_1$—2-cyclopentenones (II) with an aqueous medium.

The aqueous medium may be constituted with water alone or containing a small amount of any water-miscible or water-immiscible organic solvent (e.g. acetone, tetrahydrofuran, dioxane, toluene, xylene, diisopropyl ether, or benzene). The weight of the aqueous medium in proportion to the starting 4-hydroxy-4—$R_2$—5—$R_1$—2-cyclopentenone (II) may be ordinarily from 0.5 to 100 fold, preferably from 5 to 40 fold.

The treatment is usually effected at a temperature of about 20 to 200°C. In order to accelerate the reaction rate, however, the presence of a metal salt in the aqueous medium or the elevation of the temperature to about 120 to 200°C is preferable. In other words, the conversion can be accomplished in a short period of time either in the presence of a metal salt even if the temperature is relatively low (e.g. 20 to 120°C) or at a temperature of about 120 to 200°C even if the metal salt is not present. The pH of the aqueous medium is to be neutral, and when the movement of the pH to the acidic side by addition of the metal salt is observed, it is preferable to maintain the pH around 7 by incorporation of an appropriate amount of a basic substance into the reaction system.

Examples of the metal salt are magnesium chloride, magnesium bromide, magnesium nitrate, magnesium sulfate, maganese chloride, zinc chloride, copper chloride, copper sulfate, cobalt acetate, cobalt chloride, etc. The amount of the metal salt may be usually from 0.001 to 10 mol, preferably from 0.01 to 1 mol, per one mole of the 2—$R_1$—3—$R_2$—4-hydroxy-2-cyclopentenone (I).

The mechanism involved in the reaction of the present invention is still uncertain. However, it may be presumed that the mechanism is substantially the same as in the use of an acid catalyst for the conversion of the prostaglandin intermediate into prostaglandin but, unlike when an acid catalyst is used, the formation of undesired by-products probably resulting from the aldol condensation of the once produced 2—$R_1$—3—$R_2$—4-hydroxy-2-cyclopentenone (I) is markedly inhibited.

The starting 4-hydroxy-4—$R_2$—5—$R_1$—2-cyclopentenone (II) can be produced, for instance, by reacting a furfural of the formula:

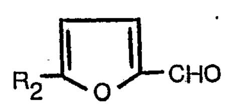

wherein $R_2$ is as defined above with an organic metal halide of the formula:

$$R_1MX$$

wherein M is Mg, Zn or Al(2/3), X is chlorine, bromine or iodine and $R_1$ is as defined above, the organic metal halide being obtainable by reacting a halide of the formula: $R_1X$ with Mg, Zn or Al, and heating the resultant furan compound of the formula:

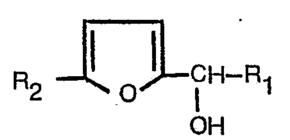

wherein $R_1$ and $R_2$ are each as defined above with water in the presence of a metal salt.

Alternatively, the 4-hydroxy-4—$R_2$—5—$R_1$—2-cyclopentenones (II) wherein $R_2$ is methyl are obtainable by the following method as disclosed in Japanese Patent Publication (unexamined) No. 21146/78:

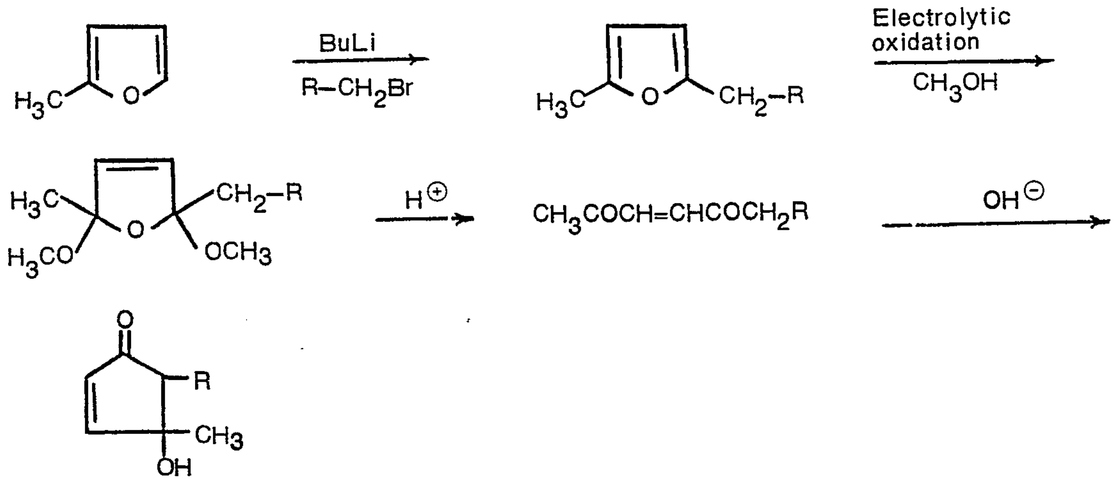


or by the following method as disclosed in G. Piancatelli et al.: Tetrahedron Letters, *39*, 3555 (1976) and ibid., *34*, 2775 (1978):

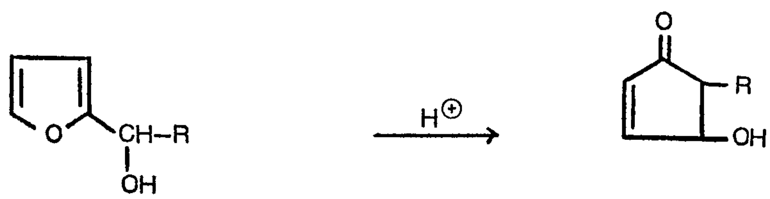


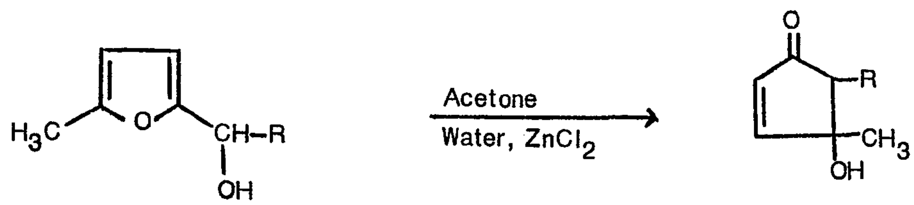


Practical and presently preferred embodiments of the present invention are illustratively shown in the following Examples.

Example 1

Into a reaction vessel a solution of 4-hydroxy-4-methyl-5-allyl-2-cyclopentenone 3 g) and $MgCl_2.6H_2O$ (4.0 g) in water (120 ml) was charged, and the temperature was elevated to 100°C. The contents were adjusted to pH 7.3 with 0.1N NaOH solution, and stirring was continued at 100°C for 4 hours, during which the pH was maintained at 7.0 to 7.3. After cooling, NaCl (40 g) was added to the reaction mixture, and the resultant mixture was extracted with ether (120 ml) 4 times. The extracts were combined together, dried over anhydrous magnesium sulfate and concentrated at 40°C under reduced pressure to give an oily substance (2.7 g). The oily substance was chromatographed with silica gel (30 g), followed by development with a solvent mixture of ethyl acetate and hexane (1:2 by volume) to give 2-allyl-3-methyl-4-hydroxy-2-cyclopentenone (2.6 g). Yield, 87%. N.M.R. ($CDCl_3$, internal standard TMS, $\delta$ ppm, 90 MHz): 5.71 (complex m, 1H, $—CH_2—CH=CH_aH_b$); 5.06 (m, 1H, $—CH_2—CH=CH_aH_b$); 4.93 (m, 1H, $—CH_2—CH=CH_aH_b$); 4.74 (broad d, 1H, 4—H); 3.94 (broad s, 1H, 4—OH); 2.96 (d, 2H, $—CH_2—CHCH_aH_b$); 2.85 (d of d, 1H, 5—H) 2.27 (d of d, 1H, 5—H); 2.11 (s, 3H, $3—CH_3$).

Example 2

Into a reaction vessel a solution of 4-hydroxy-4-methyl-5-propargyl-2-cyclopentenone (3 g) and $MgCl_2.6H_2O$ (4.0 g) in water (120 ml) was charged, and the temperature was elevated to 100°C. The contents were adjusted to pH 6.8 with 0.1N NaOH solution, and stirring was continued at 100°C for 4 hours, during which the pH was maintained at 6.8 to 7.0. After cooling, NaCl (40 g) was added to the reaction mixture and extracted with ether (120 ml) 4 times. The extracts were combined together, dried over anhydrous magnesium sulfate and concentrated at 40°C under reduced pressure to give an oily substance (2.5 g). The oily substance was chromatographed with silica gel (30 g), followed by development with a solvent mixture of ethyl acetate and hexane (1:2 by volume) to give 2-propargyl-3-methyl-4-hydroxy-2-cyclopentenone (2.3 g). Yield, 77%. N.M.R. ($CDCl_3$, internal standard TMS, $\delta$ ppm,

90 MHz): 4.60 (broad d, 1H, 4—H); 3.95 (broad s, 1H, 4—*OH*); 3.04 (d, 2H, —$CH_2$—C≡H); 2.65 (d of d, 1H, 5—H); 2.38 (d of d, 1H, 5—H); 2.20 (s, 3H, 3—$CH_3$); 1.98 (s, 1H, C≡*CH*).

Examples 3 to 18

In the same manner as in Example 1, the 4-hydroxy-4—$R_2$—5—$R_1$—2-cyclopentenone (II) (3 g) was treated to give the corresponding 2—$R_1$—3—$R_2$—4-hydroxy-2-cyclopentenone (I). The reaction conditions and the yields are shown in Table 1.

Table 1

| Example | 4-Hydroxy-4-$R_2$-5-$R_1$-2-cyclopentenone (II) | | Amount of $MgCl_2 \cdot 6H_2O$ used (g) | pH | Yield of 2-$R_1$-3-$R_2$-4-hydroxy-2-cyclopentenone (I) |
|---|---|---|---|---|---|
| | $R_1$ | $R_2$ | | | |
| 3 | Methyl | Methyl | 4.8 | 6.8—7.1 | 88 |
| 4 | Propyl | Methyl | 4.0 | 6.5—7.1 | 83 |
| 5 | Pentyl | Methyl | 3.0 | 7.0—7.2 | 83 |
| 6 | Cyclohexyl | Methyl | 3.1 | 7.0—7.1 | 86 |
| 7 | 2-cis-Pentenyl | Methyl | 3.2 | 7.0—7.4 | 90 |
| 8 | Benzyl | Methyl | 3.0 | 6.8—7.1 | 85 |
| 9 | p-Methylbenzyl | Methyl | 2.8 | 6.7—7.0 | 79 |
| 10 | p-Chlorobenzyl | Methyl | 2.6 | 6.8—7.4 | 81 |
| 11 | Methyl | Hydrogen | 4.5 | 6.9—7.2 | 87 |
| 12 | Pentyl | Hydrogen | 3.5 | 6.8—7.3 | 85 |
| 13 | Hexyl | Hydrogen | 3.5 | 7.0—7.2 | 88 |
| 14 | Cyclohexyl | Hydrogen | 3.5 | 6.7—7.1 | 91 |
| 15 | 2-cis-Pentenyl | Hydrogen | 3.5 | 6.8—7.2 | 92 |
| 16 | Benzyl | Hydrogen | 3.0 | 6.8—7.2 | 90 |
| 17 | p-Methylbenzyl | Hydrogen | 3.0 | 7.0—7.3 | 78 |
| 18 | p-Chlorobenzyl | Hydrogen | 3.0 | 6.6—7.0 | 83 |

Example 19

Into a reaction vessel a solution of 4-hydroxy-4-methyl-5-allyl-2-cyclopentenone (3 g) and $ZnCl_2$ (5 g) in water (120 ml) was charged, and the temperature was elevated to 100°C. The contents were refluxed for 15 hours while stirring. After cooling, NaCl (40 g) was added to the reaction mixture, and the resulting mixture was extracted with toluene (120 ml) 4 times. The extracts were combined together and concentrated at 50°C under reduced pressure to give an oily substance (2.5 g). The oily substance was chromatographed on silica gel (30 g), followed by development with a solvent mixture of ethyl acetate and hexane (1:2 by volume) to give 2-allyl-3-methyl-4-hydroxy-2-cyclopentenone (2.4 g). Yield, 80%.

Examples 20 to 23

In the same manner as in Example 19, 4-hydroxy-4-methyl-5-allyl-2-cyclopentenone (3 g) was treated to give 2-allyl-3-methyl-4-hydroxy-2-cyclopentenone. The reaction conditions and the yields are shown in Table 2.

Table 2

| Example | Metal salt used | Amount of metal salt used | Reaction time (hr) | Yield (%) |
|---|---|---|---|---|
| 20 | $MgCl_2 \cdot 6H_2O$ | 10 | 50 | 70 |
| 21 | $CoCl_2 \cdot 6H_2O$ | 5 | 40 | 72 |
| 22 | $CuCl_2 \cdot 2H_2O$ | 10 | 24 | 69 |
| 23 | $CuSO_4$ | 10 | 24 | 65 |

Example 24

Into an autoclave 4-hydroxy-4-methyl-5-allyl-2-cyclopentenone (20 g) and water (400 ml) were charged, and the resultant mixture was stirred at 180°C for 6 hours in nitrogen atmosphere. After cooling, sodium chloride (50 g) was added to the reaction mixture, and the resulting mixture was extracted with methyl isobutyl ketone. The extract was concentrated under reduced pressure to give 2-allyl-3-methyl-4-hydroxy-2-cyclopentenone (19 g). Yield, 95%. M.P. 130—132°C/1.2 mmHg.

Example 25

Into an autoclave 4-hydroxy-4-methyl-5-propargyl-2-cyclopentenone (3 g) and water (400 ml) were charged, and the resultant mixture was stirred at 150°C for 6 hours in nitrogen atmosphere. After cooling, sodium chloride (50 g) was added to the reaction mixture; the resulting mixture was extracted with ethyl acetate. The extract was concentrated under reduced pressure and purified by column chromatography to give 2-propargyl-3-methyl-4-hydroxy-2-cyclopentenone (2.7 g). Yield, 90%. $n_D^{21}$ 1.5148.

Examples 26 to 41

In the same manner as in Example 25, the 4-hydroxy-4—$R_2$—5—$R_1$—2-cyclopentenone (II) (3 g) was treated to give the corresponding 2—$R_1$—3—$R_2$—4-hydroxy-2-cyclopentenone (I). The reaction conditions and the yields are shown in Table 3.

TABLE 3

| Example | 4-Hydroxy-4-$R_2$-5-$R_1$-2-cyclopentenone (II) | | Reaction temperature (°C) | Yield of 2-$R_1$-3-$R_2$-4-hydroxy-2-cyclopentenone (I) |
|---|---|---|---|---|
| | $R_1$ | $R_2$ | | |
| 26 | Methyl | Methyl | 180 | 90 |
| 27 | Propyl | Methyl | 180 | 85 |
| 28 | Hexyl | Methyl | 180 | 85 |
| 29 | Cyclohexyl | Methyl | 180 | 87 |
| 30 | 2-Cyclopentenyl | Methyl | 180 | 85 |
| 31 | 2-Cyclohexenyl | Methyl | 180 | 85 |
| 32 | α-Methylallyl | Methyl | 180 | 90 |
| 33 | α-Ethylallyl | Methyl | 180 | 89 |
| 34 | 2-cis-Pentenyl | Methyl | 180 | 88 |
| 35 | α-Methylpropargyl | Methyl | 150 | 89 |
| 36 | Benzyl | Methyl | 180 | 86 |
| 37 | p-Methylbenzyl | Methyl | 180 | 80 |
| 38 | p-Chlorobenzyl | Methyl | 180 | 82 |
| 39 | Methyl | Hydrogen | 170 | 92 |
| 40 | Hexyl | Hydrogen | 170 | 88 |
| 41 | Benzyl | Hydrogen | 170 | 90 |

**Claims**

1. A process for preparing 4-hydroxy-2-cyclopentenolones of the formula I:

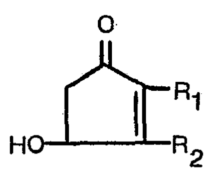

(I)

wherein $R_1$ is an alkyl group having not more than 6 carbon atoms, an alkenyl group having not more than 6 carbon atoms, an alkynyl group having not more than 6 carbon atoms or a group of the formula:

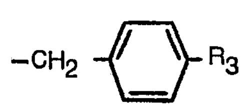

(in which $R_3$ is hydrogen, methyl or halogen) and $R_2$ is a hydrogen atom or a methyl group, provided that when $R_2$ is hydrogen, $R_1$ is neither α-methylallyl nor α-methylpropargyl, from 4-hydroxy-4—$R_2$—5—$R_1$-cyclopentenones of the formula II:

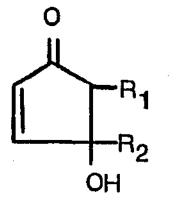

(II)

wherein $R_1$ and $R_{21}$ are each as defined above, which comprises treating a compound of formula II with an aqueous medium under roughly neutral conditions.

2. The process according to claim 1, wherein the treatment is carried out at a temperature of 20 to 200°C.

3. The process according to claim 1, wherein the treatment is carried out at a temperature of 120 to 200°C.

4. The process according to claim 1, wherein the treatment is carried out in the presence of a metal salt.

**Patentansprüche**

1. Ein Verfahren zur Herstellung von 4-Hydroxy-2-cylcopentenolonen der Formel I

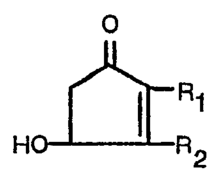

(I)

in der R1 einen Alkylrest mit nicht mehr als 6 Kohlenstoffatomen, einen Alkenylrest mit nicht mehr als 6 Kohlenstoffatomen, einen Alkynylrest mit nicht mehr als 6 Kohlenstoffatomen oder einen Rest der Formel:

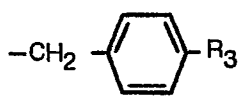

bedeutet (in der $R_3$ ein Wasserstoffatom, eine Methylgruppe oder ein Halogenatom ist) und $R_2$ ein Wasserstoffatom oder eine Methylgruppe bedeutet, mit der Maßgabe, daß $R_1$ weder $\alpha$-Methylallyl noch $\alpha$-Methylpropargyl ist, wenn $R_2$ ein Wasserstoffatom bedeutet, aus 4-Hydroxy-4—$R_2$—5—$R_1$-cyclopentenonen der Formel II:

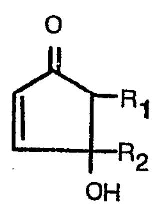

(II)

in der $R_1$ und $R_2$ die vorstehend genannte Bedeutung haben, gekennzeichnet durch Behandlung einer Verbindung der Formel II mit einem wäßrigen Medium unter ungefähr neutralen Bedingungen.

2. Das Verfahren nach Anspruch 1, wobei die Behandlung bei einer Temperatur von 20 bis 200°C durchgeführt wird.

3. Das Verfahren nach Anspruch 1, wobei die Behandlung bei einer Temperatur von 120 bis 200°C durchgeführt wird.

4. Das Verfahren nach Anspruch 1, wobei die Behandlung in Gegenwart eines Metallsalzes durchgeführt wird.

**Revendications**

1. Procédé de préparation de 4-hydroxy-2-cyclopenténolones ayant la formule I:

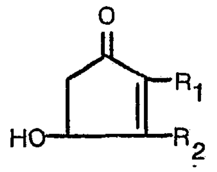

(I)

où $R_1$ est un groupe alkyle n'ayant pas plus de 6 atomes de carbone, un groupe alkényle n'ayant pas plus de 6 atomes de carbone, un groupe alkynyl n'ayant pas plus de 6 atomes de carbone ou un groupe ayant la formule:

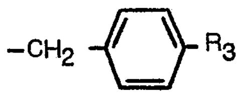

(où $R_3$ est l'hydrogène, le groupe méthyle ou un halogène) et $R_2$ est un atome d'hydrogène ou un groupe méthyle, pourvu que, quand $R_2$ est l'hydrogène, $R_1$ ne soit ni le groupe $\alpha$-méthylallyle ni le groupe $\alpha$-méthylpropargyle, à partir de 4-hydroxy-4—$R_2$—5—$R_1$-cyclopenténones ayant la formule II:

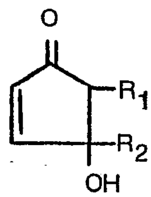

(II)

où $R_1$ et $R_2$ sont chacun comme définis ci-dessus, qui consiste à traiter un composé de formule II avec un milieu aqueux, dans des conditions à peu près neutres.

2. Procédé selon la revendication 1, dans lequel le triatement est réalisé à une température de 20 à 200°C.

3. Procédé selon la revendication 1, dans lequel le traitement est réalisé à une température de 120 à 200°C.

4. Procédé selon la revendication 1, caractérisé en ce que le traitement est réalisé en présence d'un sel métallique.